(19) **Europäisches Patentamt**
**European Patent Office**
**Office européen des brevets**

(11) Veröffentlichungsnummer: **0 139 734**
**B1**

(12) # EUROPÄISCHE PATENTSCHRIFT

(45) Veröffentlichungstag der Patentschrift:
21.02.90

(51) Int. Cl.⁵: **C 12 N 15/00, G 01 N 33/535**

(21) Anmeldenummer: **84901759.5**

(22) Anmeldetag: **19.04.84**

(86) Internationale Anmeldenummer:
**PCT/EP 84/00120**

(87) Internationale Veröffentlichungsnummer:
**WO 84/04395 (08.11.84 Gazette 84/26)**

(54) **MITTEL ZUM ANTIGEN/ANTIKÖRPER-NACHWEIS.**

(30) Priorität: **23.04.83 DE 3314812**

(43) Veröffentlichungstag der Anmeldung:
**08.05.85 Patentblatt 85/19**

(45) Bekanntmachung des Hinweises auf die Patenterteilung:
**21.02.90 Patentblatt 90/8**

(84) Benannte Vertragsstaaten:
**AT BE CH FR GB LI NL SE**

(56) Entgegenhaltungen:
**EP-A- 0 001 930**
**DE-A- 2 430 357**

**EMBO Journal Vol.1 no, 4, Seiten 509-512 1982**
**Proc. Nat. Academy of Science, USA Vol. 80, Feb.1983 S. 697-701**

**Die Akte enthält technische Angaben, die nach dem Eingang der Anmeldung eingereicht wurden und die nicht in dieser Patentschrift enthalten sind.**

(73) Patentinhaber: **Battelle-Institut e.V., Am Römerhof 35 Postfach 900 160, D-6000 Frankfurt/Main 90 (DE)**

(72) Erfinder: **HÄNGGI, Urs, Frankfurter Strasse 94, D-6238 Hofheim (DE)**
Erfinder: **MIESCHENDAHL, Martin, Burgstrasse 116, D-5060 Bergisch-Gladbach 3 (DE)**
Erfinder: **PETRI, Thomas, Bad Sodener Strasse 5, D-6231 Sulzbach (DE)**

(74) Vertreter: **Sartorius, Peter, Dipl.-Ing., Battelle-Institut e.V. Abteilung Patente Am Römerhof 35, D-6000 Frankfurt am Main 90 (DE)**

## Beschreibung

Die Erfindung betrifft ein Hybridmolekül zum Antigen/Antikörper-Nachweis auf der Basis von β-Galactosidase oder alkalischer Phosphatase und Protein A mit Antikörper-Affinität sowie ein Verfahren zu seiner Herstellung.

Immunologische Analyseverfahren gehören wegen der hohen Spezifität und der Stöchiometrie der Antigen-Antikörper-Komplexbildung zu den Standardmethoden diagnostischer Laboratorien. Bei quantitativen Analysen werden meistens der RIA-Test (radioimmunoassay) oder der ELISA-Test (enzyme-linked immunosorbent assay) eingesetzt. Im RIA-Test wird die Menge an Antigen durch Messung der Verdünnung (Verminderung) der Radioaktivität der im Test eingesetzten radioaktiv markierten Eich-Antigene durch die zu testende nicht markierte Substanz ermittelt. Im ELISA-Test erfolgt die Messung der Menge an Antigen in einem Kaskaden-Antigen-Antikörper-Komplex mit Hilfe eines enzymatischen Tests.

Die erste Methode, der RIA-Test, ist äusserst empfindlich. Sie erfordert jedoch spezielle Einrichtungen und Geräte für das Arbeiten mit radioaktiven Substanzen und bereitet darüber hinaus Schwierigkeiten mit der Entsorgung des radioaktiven Abfalls. Der ELISA-Test ist weniger empfindlich. Er ist jedoch billiger, technisch weniger aufwendig und braucht weniger Zeit.

Der ELISA-Test ist ein Mehrstufen-Verfahren. Hierbei wird ein Überschuss des gegen die Probe (Antigen) gerichteten Antikörpers (1. Antikörper) kovalent oder über hydrophobe Wechselwirkungen an einen festen Träger gebunden. Der so fixierte Antikörper wird mit der Probe inkubiert, wodurch die Antigene als Antigen-Antikörper-Komplex an den Träger gebunden werden. Dieser trägergebundene Komplex wird erneut mit antigenspezifischem, aber ungebundenem Antikörper inkubiert (2. Antikörper). Dabei wird eine der Antigenkonzentration entsprechende Menge an freiem Antikörper an den trägerfixierten Komplex gebunden. Nach dem Auswaschen des Überschusses wird der Komplex mit einem dritten, enzymkonjugierten, gruppenspezifischen Antikörper inkubiert, der gegen den konstanten Teil der schweren Kette des nichtfixierten Antikörpers ($F_c$-Teil) gerichtet ist (enzymkonjugierter, $F_c$-spezifischer Antikörper; Marker-Antikörper). Die Menge an gebundenem Marker-Antikörper wird schliesslich mit Hilfe eines enzymatischen Tests gemessen. In einer qualitativen Variante des ELISA-Tests wird das Antigen direkt an den Träger gebunden.

Merkmal des ELISA-Tests ist der enzymatische Nachweis des Antigen-Antikörper-Komplexes mit Hilfe eines an den Marker-Antikörper gebundenen Enzyms. Die Bindung des Marker-Enzyms an den Antikörper erfolgt durch kovalente Verknüpfung der beiden Proteinmoleküle. Die Verknüpfung ist unspezifisch, d.h. es ist nicht vorauszusehen, welche Bereiche des Enzyms mit welchen Bereichen des Antikörpers verknüpft werden. Es lässt sich daher auch nicht vermeiden, dass bei der chemischen Konjugation sowohl ein Teil des zur Verfügung stehenden Antikörpers als auch ein Teil des zu verknüpfenden Enzyms inaktiviert werden. Die inaktiven Konjugate sind in der Regel von den aktiven nicht zu trennen und vermindern damit die Komplexbildung und das Signal im ELISA-Test.

Aus der DE-OS 2 420 357 ist ein Verfahren zur Bestimmung von Immunoglobulin oder Antigenen bekannt, bei dem mit einem Enzym chemisch gebundenes Protein A eingesetzt wird. Es wird auch angegeben, dass zur Herstellung dieses Marker-Enzyms auch Teile von Protein A herangezogen werden können. Dieses Marker-Enzym weist jedoch ebenfalls die Nachteile der im ELISA-Test bereits verwendeten Mittel auf, insbesondere in bezug auf die unspezifische Verknüpfung des Enzyms mit Protein A.

Die EP-A 0 001 930 betrifft ein Mittel zum Antigen-Antikörper-Nachweis, bestehend aus einem radioaktiv oder mit einem Enzym markierten Protein A, wobei wiederum eine unspezifische Verknüpfung mit dem Protein A erforderlich ist. Aus The EMBO Journal Vol. 1, No. 4, Seiten 509–512, 1982, ist eine gentechnisch hergestellte Antigen/β-Galactosidase bekannt, mit der sich jeweils jedoch nur ein Antikörper erkennen lässt; für andere Antikörper muss eine neue Antigen/β-Galactosidase konstruiert werden. Schliesslich beschreibt Proc. Natl. Acad. Sci. USA, Vol. 80, February 1983, Seiten 697–701, die Herstellung von Protein A auf genetischem Wege unter Verwendung des isolierten Gens für Protein A.

Der Erfindung liegt die Aufgabe zugrunde, ein Mittel bereitzustellen, mit dem sich das Problem der inaktiven Konjugate vermeiden lässt und die Verknüpfung Marker-Enzym-Antikörper nicht ungerichtet, sondern gezielt erfolgt.

Diese Aufgabe wird erfindungsgemäss mit einem Hybridmolekül zum Antigen/Antikörper-Nachweis auf der Basis eines Enzyms, das mit einem antikörperbindenden Protein auf genetischem oder gentechnischem Wege fusioniert ist, gelöst, bei dem das Enzym β-Galactosidase oder alkalische Phosphatase und das antikörperbindende Protein Protein A ist.

Gemäss einer vorteilhaften Ausführungsform der Erfindung ist β-Galactosidase oder alkalische Phosphatase mit einem antikörperbindenden Abschnitt des Proteins A fusioniert.

Die Verfahrensansprüche 3 bis 6 betreffen vorteilhafte Merkmale für die Herstellung der Hybridmoleküle der Erfindung.

Erfindungsgemäss wird der enzymkonjugierte, Fc-spezifische Antikörper des ELISA-Tests durch ein Fusionsprotein ersetzt. Zur Herstellung des Fusionsproteins wird das antikörperbindende Protein A aus Staphylococcus aureus, Staphylococcus epidermis oder anderen Bakterienarten, in nichtessentielle Teile von Enzymen eingebaut. Vorzugsweise wird nur ein antikörperbindender Abschnitt des Proteins mit dem Enzym verbunden. Der Enzym-Anteil des Fusionsproteins besteht aus den an sich bekannten, bei ELISA-Test einsetzbaren Enzymen β-Galactosidase oder alkalische Phosphatase. Die Fusion des Enzyms mit dem antikörperbindenen Protein erfolgt vorzugsweise analog zu den an sich bekannten genetischen oder gentechnischen Methoden. Das erhaltene Hybrid-Molekül erhöht ohne Einbusse der Spezifität wegen des Wegfalls der inaktiven Konjugate die Sensibilität des Tests.

Die Erfindung wird anhand der beiliegenden Zeichnung näher erläutert. Es zeigen

Fig. 1 Herstellung des erfindungsgemässen Hybrid-Moleküls aus Protein A und β-Galaktosidase;

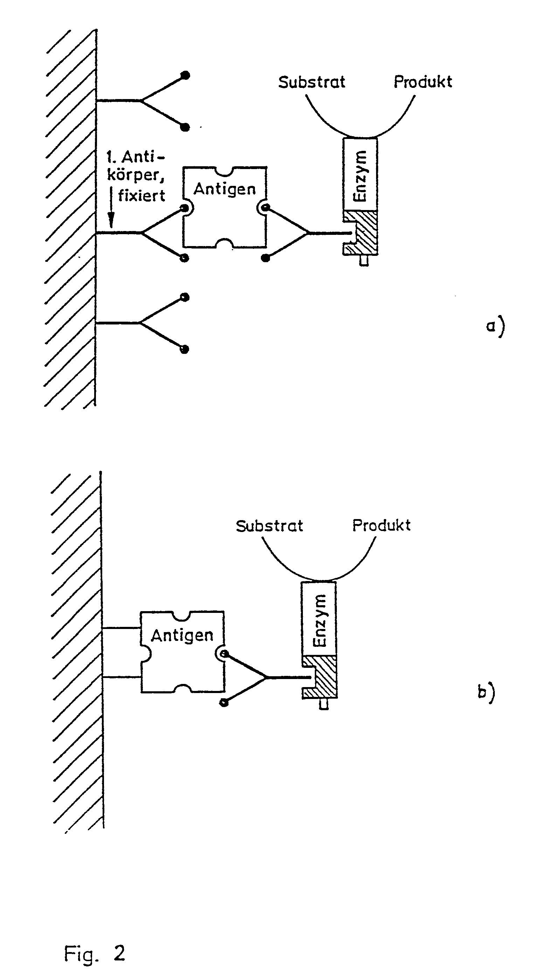

Fig. 2 a) und b) den Antikörper/Antigen-Nachweis mit dem erfindungsgemässen Hybrid-Molekül und

Fig. 3 Identifizierung von Klonen, welche $F_c$-bindende Galaktosidase produzieren.

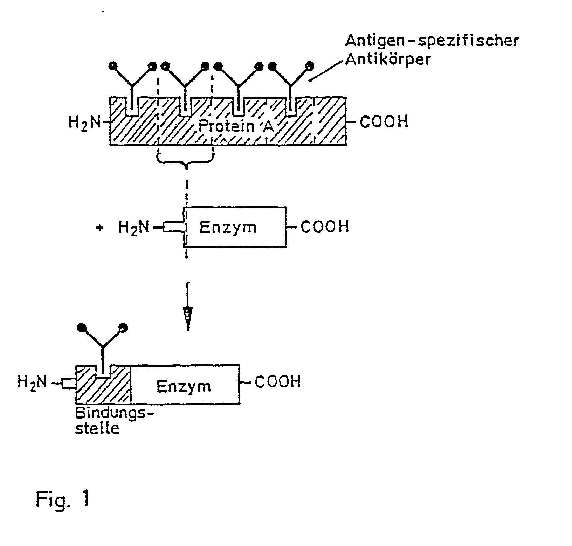

Vorzugsweise wird ein $F_c$-spezifischer Abschnitt des Proteins A aus Staphylococcus aureus mit β-Galaktosidase verbunden. Dadurch entsteht ein funktionsfähiges Enzym-Molekül mit einer hohen Affinität zum $F_c$-Teil von Antikörpern (Fig. 1). Dieses Hybrid-Molekül ersetzt den dritten Antikörper in ELISA-Test.

Protein A wird verwendet, weil es ein Antikörper-Protein mit hoher Affinität gegen den $F_c$-Teil von IgG-Molekülen verschiedener Spezies ist und mehrere identische oder doch sehr ähnliche Bindungsstellen besitzt. Bei der Konstruktion des erfindungsgemässen Marker-Antikörper-Ersatzes muss daher nicht das ganze Gen kloniert werden. Es genügt, jenen Teil zu finden, der für eine der Bindungsstellen kodiert. Die Bindung von Protein A an die Antikörper verändert die Antigen-Bindungsstellen nicht, so dass Antikörper mit gebundenem Protein A einen normalen Antigen-Antikörper-Komplex eingehen können.

β-Galaktosidase wird als Enzym bevorzugt, weil die Spezifität des Enzyms bei der Zuckerkomponente des Glykosids liegt. Das Enzym spaltet daher Galaktoside mit unterschiedlichen Alkoholkomponenten, was in empfindlichen chromogonen Aktivitätstests ausgenutzt werden kann. Darüber hinaus ist der Amino-Terminus des Enzyms für die Aktivität nicht essentiell. Er kann ohne Verlust der Aktivität deletiert oder substituiert werden. Diese Eigenschaft wird in der Molekularbiologie häufig zur Messung der Aktivität von genetischen Regulationselementen über Fusionsproteinen benutzt.

Die Konstruktion einer $F_c$-bindenden Galaktosidase kann analog zu den bekannten genetischen bzw. gentechnischen Methoden in mehreren Stufen erfolgen. hierzu werden z. B. aus Protein A produzierenden Staphylococcen die DNS isoliert. Die DNS wird in Stücke von ca. 3000–5000 Basenpaaren Länge (entspricht etwa der Grösse einer Bindungsstelle) gespalten. Die DNA-Fragmente werden im 5'-Teil des Galaktosidase-Gens (entspricht dem nichtessentiellen Teil des Enzyms) kloniert. Für das Screening der Klone werden Antikörper-Moleküle unspezifisch an Filter gebunden und mit lysierten Bakterienklonen inkubiert.

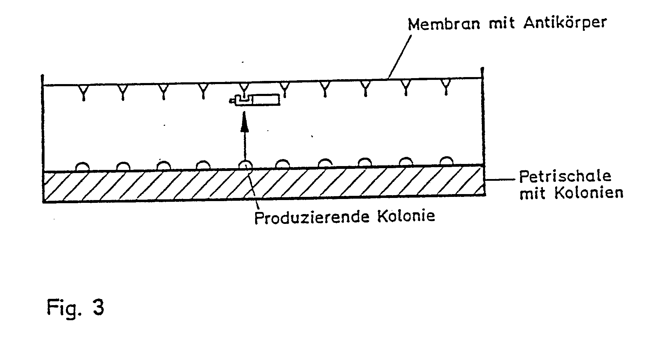

Wenn einer der Klone Antikörper-bindende Galaktosidase produziert, bleiben die Enzym-Moleküle über die Bindungsstellen am Filter hängen und können über die Enzym-Reaktion leicht nachgewiesen werden (Fig. 3).

## Patentansprüche für die Vertragsstaaten BE CH FR GB LI NL SE

1. Hybridmolekül zum Antigen/Antikörper-Nachweis auf der Basis eines Enzyms, das mit einem antikörperbindenden Protein auf genetischem oder gentechnischem Wege fusioniert ist, dadurch gekennzeichnet, dass das Enzym β-Galactosidase oder alkalische Phosphatase und das antikörperbindende Protein Protein A ist.

2. Hybridmolekül nach Anspruch 1, dadurch gekennzeichnet, dass β-Galactosidase oder alkalische Phosphatase mit einem antikörperbindenden Abschnitt des Proteins A fusioniert ist.

3. Verfahren zur Herstellung eines Hybridmoleküls nach einem der Ansprüche 1 oder 2, dadurch gekennzeichnet, dass man das Enzym-Gen der β-Galactosidase bzw. der alkalischen Phosphatase in einem für die Enzymaktivität nicht essentiellen Bereich öffnet, die DNS von antikörperbindendes Protein A produzierendem Staphylococcus aureus isoliert und spaltet sowie die Spaltstücke im geöffneten Enzym-Gen kloniert, wobei man anschliessend eine Screening der Klone vornimmt.

4. Verfahren nach Anspruch 3, dadurch gekennzeichnet, dass man die gesamte genetische Information des antikörperbindenden Proteins A oder nur solche des antikörperbindenden Bereichs enthaltende DNS-Spaltstücke verwendet.

5. Verfahren nach Anspruch 3 oder 4, dadurch gekennzeichnet, dass man zum Screening der Klone die lysierten Bakterien an antikörperbedeckte Filter überträgt und die spezifisch gebundenen Enzymmoleküle mit Hilfe eines enzymatischen Tests identifiziert.

6. Verfahren nach mindestens einem der Ansprüche 3 bis 5, dadurch gekennzeichnet, dass man die DNS von Staphylococcus aureus zu Bruchstücken mit einer Länge zwischen 1 000 bis 10 000, vorzugsweise 3000 bis 5000 Nukleotiden spaltet.

## Patentansprüche für den Vertragsstaat AT

1. Verfahren zur Herstellung eines Hybridmoleküls zum Antigen/Antikörper-Nachweis auf der Basis eines Enzyms, das mit einem antikörperbindenden Protein auf genetischem oder gentechnischem Wege fusioniert ist, dadurch gekennzeichnet, dass man das Enzym-Gen der β-Galactosidase bzw. der alkalischen Phosphatase in einem für die Enzymaktivität nicht essentiellen Bereich öffnet, die DNS von antikörperbindendes Protein A produzierendem Stpahylococcus aureus isoliert und spaltet sowie die Spaltstücke im geöffneten Enzym-Gen kloniert, wobei man anschliessend ein Screening der Klone vornimmt.

2. Verfahren nach Anspruch 1, dadurch gekennzeichnet, dass man die gesamte genetische Information des antikörperbindenden Proteins A oder nur solche des antikörperbindenden Bereichs enthaltende DNS-Spaltstücke verwendet.

3. Verfahren nach Anspruch 1 oder 2, dadurch gekennzeichnet, dass man zum Screening der Klone die lysierten Bakterien an antikörperbedeckte Filter überträgt und die spezifisch gebundenen Enzymmoleküle mit Hilfe eines enzymatischen Tests identifiziert.

4. Verfahren nach mindestens einem der Ansprüche 1 bis 3, dadurch gekennzeichnet, dass man die DNS von Staphylococcus aureus zu Bruchstücken mit einer Länge zwischen 1000 bis 10000, vorzugsweise 3000 bis 5000 Nukelotiden spaltet.

## Claims for the Contracting States BE CH FR GB LI NL SE

1. Hybrid molecule for antigen/antibody detection on the basis of an enzyme fused with an antibody-binding protein either genetically or by genetic engineering, comprising: the enzyme being β-galactosidase or alkaline phosphatase, and the antibody-binding protein being protein A.

2. Hybrid molecule as claimed in claim 1, wherein β-galactosidase or alkaline phosphatase is fused with an antibody-binding part of protein A.

3. Method of producing a hybrid molecule as claimed in any of the claims 1 or 2, wherein the enzyme gene of β-galactosidase or of alkaline phosphatase is opened in a region that is not essential for enzyme activity, the DNA of Staphylococcus aureus that produces antibody-binding protein A is isolated and cleaved, and the cleavage fragments are cloned in the opened enzyme gene, the clones being subsequently screened.

4. Method as claimed in claim 3, wherein the total genetic information of the antibody-binding protein A is used, or only those DNA cleavage fragments are used which contain the antibody-binding regions.

5. Method as claimed in claim 3 or claim 4, wherein, in order to screen the clones, the lysed bacteria are transferred to antibody-covered filters, and the specifically bound enzyme molecules are identified by means of an enzymatic test.

6. Method as claimed in at least one of claims 3 to 5, wherein the DNA of Staphylococcus aureus is cleaved into fragments having a length between 1,000 and 10,000, preferably between 3,000 and 5,000, nucleotides.

## Claims for the Contracting State AT

1. Method of producing a hybrid molecule for antigen/antibody detection on the basis of an enzyme fused with an antibody-binding protein either genetically or by genetic engineering, comprising: opening the enzyme gene of β-galactosidase or of alkaline phosphatase in a region that is not essential for enzyme activity, isolating and cleaving the DNA of Staphylococcus aureus that produces antibody-binding protein A, and cloning the cleavage fragments in the opened enzyme gene, the clones being subsequently screened.

2. Method as claimed in claim 1, wherein the total genetic information of the antibody-binding protein A is used, or only those DNA cleavage fragments are used which contain the antibody-binding regions.

3. Method as claimed in claim 1 or claim 2, wherein, in order to screen the clones, the lysed bacteria are transferred to antibody-covered filters, and the specifically bound enzyme molecules are identified by means of an enzymatic test.

4. Method as claimed in at least one of the claims 1 to 3, wherein the DNA of Staphylococcus aureus is cleaved into fragments having a length between 1,000 and 10,000, preferably between 3,000 and 5,000, nucleotides.

## Revendications pour les Etats contractants BE CH FR GB LI NL SE

1. Molécule hybride pour la détermination d'anticorps-antigène, à base d'une enzyme qui est fusionnée par voie génétique ou de génie génétique avec une protéine se fixant à un anticorps, caractérisée en ce que l'enzyme est la β-galactosidase ou la phosphatase alcaline et la protéine se fixant à un anticorps est la protéine A.

2. Molécule hybride selon la revendication 1, caractérisée en ce que la β-galactosidase ou la phosphatase alcaline est fusionnée avec un fragment de la protéine A se fixant à un anticorps.

3. Procédé pour la préparation d'une molécule hybride selon l'une des revendications 1 ou 2, caractérisé en ce que l'on ouvre le gène d'enzyme de la β-galactosidase ou de la phosphatase alcaline en une région non essentielle pour l'activité enzymatique, on isole et coupe l'ADN de staphylococcus aureus producteur de protéine A se fixant à un anticorps, et on clone les fragments de coupure dans le gène ouvert de l'enzyme, en effectuant ensuite un criblage des clones.

4. Procédé selon la revendication 3, caractérisé en ce que l'on utilise l'information génétique totale de la protéine A se fixant à un anticorps ou seulement les fragments d'ADN coupés qui contiennent la région se fixant à un anticorps.

5. Procédé selon la revendication 3 ou 4, caractérisé en ce que, pour le criblage des clones, on transfère sur filtre couvert d'anticorps les bactéries lysées et on identifie à l'aide d'un essai enzymatique les molécules d'enzyme liées spécifiquement.

6. Procédé selon au moins l'une quelconque des revendications 3 à 5, caractérisé en ce que l'on coupe l'ADN de staphylococcus aureus en fragments ayant une longueur comprise entre 1000 et 10000, de préférence entre 3000 et 5000 nucléotides.

**Revendications pour l'Etat contractant AT**

1. Procédé pour la préparation d'une molécule hybride pour la détermination d'anticorps-antigène, à base d'une enzyme qui est fusionnée par voie génétique ou de génie génétique avec une protéine se fixant à un anticorps, caractérisé en ce que l'on ouvre le gène d'enzyme de la β-galactosidase ou de la phosphatase alcaline dans une région non essentielle pour l'activité enzymatique, on isole et coupe l'ADN de staphylococcus aureus producteur de protéine A se fixant à un anticorps, et on clone les fragments de coupure dans le gène d'enzyme ouvert, en effectuant ensuite un criblage des clones.

2. Procédé selon la revendication 1, caractérisé en ce que l'on utilise l'information génétique totale de la protéine A se fixant à un anticorps ou seulement les fragments d'ADN coupés qui contiennent la région se fixant à un anticorps.

3. Procédé selon la revendication 1 ou 2, caractérisé en ce que pour le criblage des clones, on transfère sur filtre couvert d'anticorps les bactéries lysées et on identifie à l'aide d'un essai enzymatique les molécules d'enzyme liées spécifiquement.

4. Procédé selon au moins l'une quelconque des revendications 1 à 3, caractérisé en ce que l'on coupe l'ADN de staphylococcus aureus en fragments ayant une longueur comprise entre 1000 et 10 000, de préférence entre 3000 et 5000 nucléotides.

Fig. 1

Fig. 2

Membran mit Antikörper

Petrischale
mit Kolonien

Produzierende Kolonie

Fig. 3